# EUROPEAN PATENT SPECIFICATION

(11) **EP 1 148 123 B1**
(45) Date of publication and mention of the grant of the patent: **11.05.2005**
(21) Application number: 00902089.2
(22) Date of filing: 04.02.2000
(51) Int. Cl.: C12N 15/09, G06F 17/50, G06F 19/00

(54) **METHOD FOR DETERMINING BASE SEQUENCE OF ANALYTICAL OLIGONUCLEOTIDES FOR THE DETECTION OF NUCLEIC ACIDS**
VERFAHREN ZUR BESTIMMUNG DER BASENSEQUENZ ANALYTISCHER OLIGONUKLEOTIDE ZUM NACHWEIS VON NUKLEINSÄUREN
PROCEDE PERMETTANT DE DETERMINER UNE SEQUENCE DE BASE D'UNE OLIGONUCLEOTIDE ANALYTIQUE POUR LA DETECTION DES ACIDES NUCLEIQUES

(30) Priority: 04.02.1999 JP 2773699
(43) Date of publication of application: 24.10.2001
(73) Proprietor: Olympus Corporation, Tokyo 151-0072 (JP)
(72) Inventor: SUYAMA, Akira, Hachioji-shi, Tokyo 192-0372 (JP)
(74) Representative: Kaiser, Jürgen, Dr.rer.nat.Dipl.-Chem.
(86) International application number: PCT/JP2000/000610
(87) International publication number: WO 2000/046363

(56) References cited:
- BECKER A ET AL: "Primer Design - a new program to choose PCR primers and oligonucleotide probes" MEDIZINISCHE GENETIK, BERUFSVERBAND MEDIZINISCHE GENETIK, MUNCHEN,, DE, vol. 7, no. 2, 1995, pages A-158, XP002152451 ISSN: 0936-5931
- HYNDMAN D ET AL: "Software to determine optimal oligonucleotide sequences based on hybridization simulation data." BIOTECHNIQUES, vol. 20, no. 6, 1996, pages 1090-1094, 1096-1097, XP002932984 ISSN: 0736-6205
- MITSUHASHI M ET AL: "OLIGONUCLEOTIDE PROBE DESIGN - A NEW APPROACH" NATURE, MACMILLAN JOURNALS LTD. LONDON, GB, vol. 367, 24 February 1994 (1994-02-24), pages 759-761, XP002948495 ISSN: 0028-0836
- G. PESOLE ET AL.: 'GeneUp: a program to select short PCR primer paris that occur in multiple members of sequence lists' BIOTECHNIQUES, vol. 25, no. 1, 1998, pages 112 - 123, XP002927689
- P.A. ZIMMERMAN ET AL.: 'Optimizing probe selection in directed heteroduplex analysis using HDProbe 1.1' BIOTECHNIQUES, vol. 19, no. 6, 1995, pages 972 - 977, XP002927690
- EUGENE W. MYERS ET AL.: 'Computer program for the IBM personal computer which searches for approximate matches to short oligonucleotide sequences in long target DNA sequences' NUCLEIC ACIDS RESEARCH, vol. 14, no. 1, 1986, pages 501 - 508, XP002927691
- AKIRA TOYAMA.: 'Oligonucleotide wo mochiita sequencing ho' TANPAKUSHITSU KAKUSAN KOSO, vol. 38, no. 3, 1993, pages 647 - 657, XP002946267

## Description

### Technical Field

The present invention relates to a method of determining a nucleotide sequence of an analytical oligo nucleic acid for analytical use (referred to as an analytical oligo nucleic acid, hereinafter), which is used to detect the nucleotide sequence of a specific nucleic acid. More specifically, the present invention relates to a method of quickly and efficiently determining the nucleotide sequence of the analytical oligo nucleic acid which can detect a predetermined partial nucleotide sequence of an extremely long nucleotide sequence of a nucleic acid. Therefore, the method has effective utility in the determination of nucleotide sequences for nucleic acid analysis.

In the method of the present invention, the nucleotide sequence of a desired analytical oligo nucleic acid can be simply and efficiently determined by combining a plurality of calculation processes which can be carried out by an apparatus having a limited calculation capacity.

### Background Art

In general, in a double-stranded chain in which nucleic acids form a hybrid, when a pair of bases facing each other do not satisfy the base pairing according to Watson-Crick base pair (adenine-thymine or uracil, or cytosine-guanine), it is said that a mis-hybridization occurs or a mismatch takes place. If a mismatch occurs, the thermal stability of the hybrid generally decreases. Thus, the mismatch can be avoided by raising the hybridization temperature. However, if a hybrid is formed with the analytical oligo nucleic acid of 30-nucleotide long and the formed hybrid has a single mismatch at the 3' terminal or the 5' terminal, its thermal stability is almost the same as that of the completely-matched hybrid. In this case, even if the hybridization temperature is raised, it is difficult to distinguish a mismatched hybrid from a matched hybrid. Therefore, when such a mis-hybridization takes place, false detection will result.

To overcome the mismatch, all possible structures assumed by hybrids formed of the nucleotide sequence of a designed analytical oligo nucleic acid and a target nucleic acid to be detected are required to be deduced through calculation, and further, it is required to demonstrate that the nucleotide sequence of the designed analytical oligo nucleic acid would not form a mismatch as mentioned above. However, it takes a long time to deduce all the possible structures through calculation. Therefore, in a conventional designing for the nucleotide sequence of the analytical oligo nucleic acid, it has not been evaluated in advance how strong the designed candidate sequence specifically hybridizes with a target site. As a result, even if the structure is deduced by spending a lot of time on calculation, good results are not obtained. Therefore, the candidate sequences obtained after elaborate work are obliged to be discarded in many cases.

Detecting the nucleotide sequence of a specific nucleic acid present in a biological sample is not only important in analyzing a protein which is expressed and functioning in a specific organ at a molecular level, and thereby studying expression control of a protein during information transmission in the nervous, brain or immune system, but also an important technique for gene diagnosis used for detecting mutant genes in genetic diseases, diagnosing cancers, and detecting virus-associated genes. Particularly, the gene diagnosis is used for final diagnosis where no mistakes are allowed. Furthermore, in the field called "molecular computing", when performing the operation of combinatorial problem using DNAs, the nucleotide sequence of a nucleic acid has to be accurately detected in order to verify what solution has been obtained.

In gene analysis for analyzing the presence or absence of the gene and mutation on the basis of qualitative and quantitative detection of various types of nucleic acid molecules, the most important is to determine a nucleotide sequence of the oligo nucleic acid for use in analysis (hereinafter referred to as "analytical sequence") which forms a double strand specifically with only a predetermined site of a target nucleic acid. In a nucleic acid hybridization reaction, mis-hybridization is likely to occur in the case where the employed analytical sequence is analogous to a complementary sequence at any site except for the target site of the gene to be detected. Therefore, the analytical sequence tends to be designed so as to have a length as long as possible in order to improve the specificity to the target sequence. However, the longer the probe sequence, the more stable the secondary structure of the analytical oligo nucleic acid itself. As a result, hybridization efficiency of the probe with the target nucleic acid significantly decreases and a hybridization temperature increases. As a consequence, the hybridization reaction will be complicated.

Furthermore, much experience, and trial and error are required to select the analytical sequence. In addition, a conventional calculation method for determining a probe sequence having a stringent specificity requires enormously amount of time for calculation. Under these circumstances, it has been increasingly and strongly demanded that the analytical sequence be easily designed based only upon calculation without depending upon experience and without performing numerous preliminary experiments.

### Disclosure of Invention

A first object of the present invention is to provide a method of determining a nucleotide sequence of an analytical oligo nucleic acid, namely, an analytical sequence having a high specificity and capable of always performing a highly efficient hybridization reaction.

A second object of the present invention is to provide a method of rapidly determining an analytical sequence having a high specificity.

A third object of the present invention is to rapidly and economically provide a desired analytical sequence by using a simple apparatus such as a personal computer.

The aforementioned objects are attained by a method of determining a nucleotide sequence of an analytical oligo nucleic acid for use in analysis of the nucleic acid, comprising:
listing all unit nucleotide sequences present on a target nucleic acid to be analyzed and having a predetermined length which is shorter than the analytical oligo nucleic acid to be designed; and
extracting a nucleotide sequence containing a sequence occurring at a low frequency on the target nucleic acid from the candidate sequences of the analytical oligo nucleic acids, as an analytical sequence suitable for analysis for the nucleotide sequence of the target nucleic acid, on the basis of occurrence frequency of the individual unit sequences listed.

It is preferable that the extraction step be performed by successively applying a plurality of different processing procedures.

It is preferable that the extraction step further comprises a step of selecting candidate sequences on the basis of chemical properties of individual candidate sequences. In this case, the sequence can be effectively determined since selection is made on the basis whether the probe sequence is suitable or not for hybridization reaction. In particular, if the thermal stability of a molecular structure is employed as the chemical property for the selection criteria, selection can be made depending upon suitability for the hybridization reaction. As the chemical property for the selection criteria, either thermal stability of a double strand formed of the candidate sequence or the stability of a secondary structure of the candidate sequence, or both are preferable.

Furthermore, in the present invention, there is provided a method of determining a nucleotide sequence for use in detecting a nucleic acid sequence, comprising:
a first calculation step of calculating the occurrence frequency of each of n unit sequences (hereinafter referred to as "n unit sequence") formed of n number of nucleotides (n is an integer of 2 or more) occurring on a nucleotide sequence of a known nucleic acid, on the basis of 4ⁿ types which correspond to all of the n unit sequences;
a first extraction step of extracting, from p unit sequences formed of p number of nucleotides (p is larger than n by m; and m is an integer of 1 or more), any p unit sequences present on the target nucleic acid to be analyzed;
a second calculation step of calculating a occurrence frequency index of each of the p unit sequences present on the target nucleic acid to be analyzed, on the basis of the occurrence frequency of the n unit sequences obtained in the first calculating step; and
a second extraction step of extracting, as the probe sequence, a p unit sequence having a lower occurrence frequency index obtained in the second calculation step.

In the first calculation step, it is preferable that n be any one of 5, 6, and 7. In this case, all types of n sequences which are the bases for obtaining frequencies are 1024 for n=5, 4096 for n=6, 16384 for n=7. These figures are acceptable for the first calculation step, which enables the calculation to be performed at a practical processing speed. The length of each of the p unit sequences in the first extraction step can be set at any value sufficiently to synthesize a nucleic acid probe, for example, within p=10-50.

Furthermore, it is preferable that, in at least the second extraction step, the p unit sequence for the analytical sequence be selected from a plurality of p unit sequences having a low occurrence frequency, taking chemical conditions into consideration. As the chemical conditions, the stability of a molecular structure is preferably used, and a Tm value and/or a stability of an intramolecular secondary structure are more preferably used. When both the Tm value and the stability of a secondary structure are used, it is preferable that the selection step is performed by first selecting a plurality of p unit sequences having a Tm value of a predetermined range, and then further selecting the p unit sequences having an unstable secondary structure from the p unit sequences selected on the basis of the Tm value.

Furthermore, the amount of calculation performed in the sequence determination method described above is relatively low. Therefore, if all steps are sequentially performed by a computer, it is possible to determine an analytical sequence easily and at a low cost. In this case, ultra speed calculation as that of supercomputer is not required, and thus, an advantage is obtained that a generally-used personal computer can be used.

The stability of the secondary structure may be used as an indication for determining whether or not the nucleic acid molecule makes an intramolecular hybrid within the nucleic acid molecule itself. If the nucleic acid probe forms a stable secondary structure within the molecule itself, it is difficult to form a desired hybrid between the probe and a target nucleic acid. The stable secondary structure used herein includes a loop formed of a nucleic acid and partial hybridization of the probe nucleic acid molecules with each other. The nucleic acid forming no stable secondary structure efficiently binds to the sequence of a target nucleic acid when the target nucleotide sequence is analyzed.

The probe sequence obtained in the present invention is used not only to detect the nucleic acid sequence of a gene but also to detect a nucleic acid having an artificially synthesized sequence and a partial sequence. More specifically, the probe sequence can be used to detect a specific nucleotide sequence of the artificially synthesized nucleic acid, to detect a specific cDNA included in a cDNA library, or to detect a sequence of an exon portion in a genomic sequence of eukaryote. Furthermore, it is possible to detect not only a nucleotide sequence in a genomic DNA of a living organism and a nucleotide sequence of a messenger RNA, but also copies thereof and partial sequences thereof. Moreover, the method of the present invention can be used to design a probe sequence for various enzyme reactions using a hybridization reaction of a nucleic acid, such as the primer used in PCR (Polymerase Chain Reaction).

### Brief Description of Drawings

FIG. 1 is a flow chart schematically showing a procedure according to the method of the present invention;
FIG. 2 is a schematic view showing how to set a tuple as a unit sequence;
FIG. 3 is a schematic view showing how to set a primary candidate for an analytical sequence;
FIG. 4 is a graph showing a distribution of occurrence frequency of the unit sequence on a nucleic acid;
FIG. 5 is a graph showing a distribution of Tm values of candidates for the analytical oligo nucleic acid, calculated on the basis of the nucleotide sequences; and
FIG. 6 is a schematic view showing possible shapes of the analytical oligo nucleic acids under hybridization conditions.

### Best Mode for Carrying Out of the Invention

The method of the present invention will be explained with reference to the accompanying drawings. However, the present invention will not be limited by the following explanation.

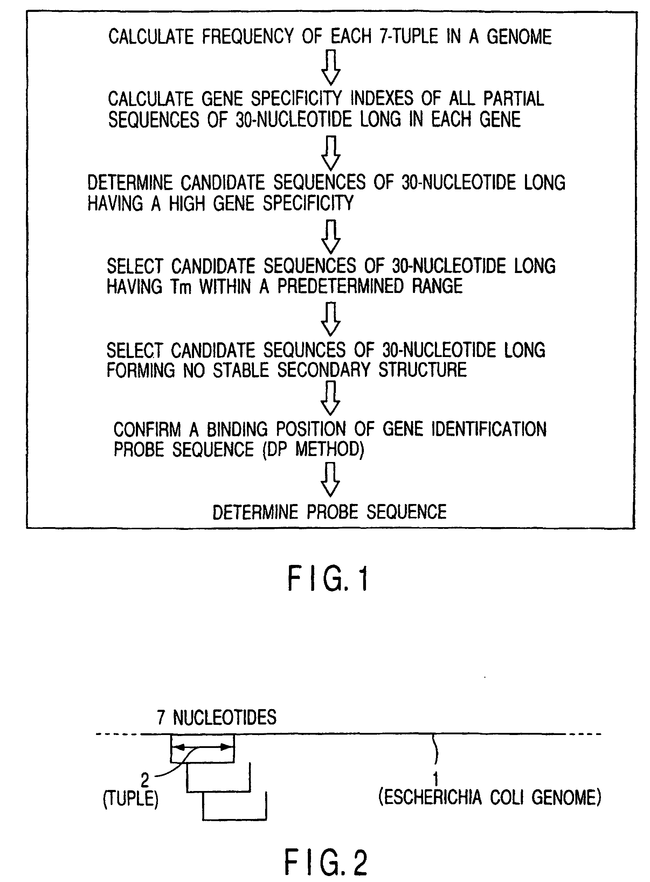

FIG. 1 is a flow chart schematically showing an embodiment of the method according to the present invention. More specifically, FIG. 1 shows the steps of designing a nucleotide sequence of a probe nucleic acid serving as an analytical oligo nucleic acid for use in gene identification. In the embodiment, a probe nucleic acid is designed for detecting a specific ORF (Open Reading Frame) on the gemone of an Escherichia coli (E. coli). Prokaryote such as E. coli does not have an Exon/Intron structure although eukaryote has. Therefore, most of ORFs of the prokaryote correspond the nucleotide sequence of a gene. To be more specific, detecting a specific ORF means detecting a specific gene. In the embodiment, there is provided a high-speed algorithm in which a calculation amount increases in proportion to the length of a genome.

First, the nucleotide sequence of the entire genome of E. coli is scanned to completely list all unit sequences each constituted of 7 nucleotides (hereinafter, referred to as "7 tuple") present in the genome. For example, as shown in FIG. 2, the nucleotide sequence consisting of first to seventh nucleotides from an appropriate terminal (sequence) of Genome 1 is determined as a first tuple 2. Thereafter, the frame consisting of the first 7-tuple 2 is shifted by one nucleotide on the genome to obtain the second 7-tuple, the third 7-tuple, the fourth 7-tuple and so on. If the procedure is sequentially repeated, all 7 tuples can be completely listed. Subsequently, all the 7 tuples are classified into types based on the nucleotide sequences thereof, and then, all kinds of 7 tuples are checked as to how many number of each of 7-tuples are present on the genome.

Next, the all kinds of 7 tuples classified based on individual nucleotide sequences are checked for the occurrence frequency thereof. If the frequency is regarded to be an existence rate, the total number of 7 tuples existing on Genome 1 has to be employed as a denominator. However, the sum of rates of existence is not necessary to reach 100% in the present invention. It is sufficient if relative frequencies of different 7 tuples occurring on the genome are obtained. For this reason, it is practical to employ the number of mathematically possible 7-tuple combinations as the denominator, for convenience sake. To explain more specifically, since a nucleotide sequence of a gene is constituted of four types of nucleotide-bases (adenine, thymine, guanine, and cytosine), the types of nucleotide sequences possibly constituting the 7 tuples will be 4⁷(=16384) in theory. In more general, the number of types will be 4ⁿ when an n-tuple unit is used.

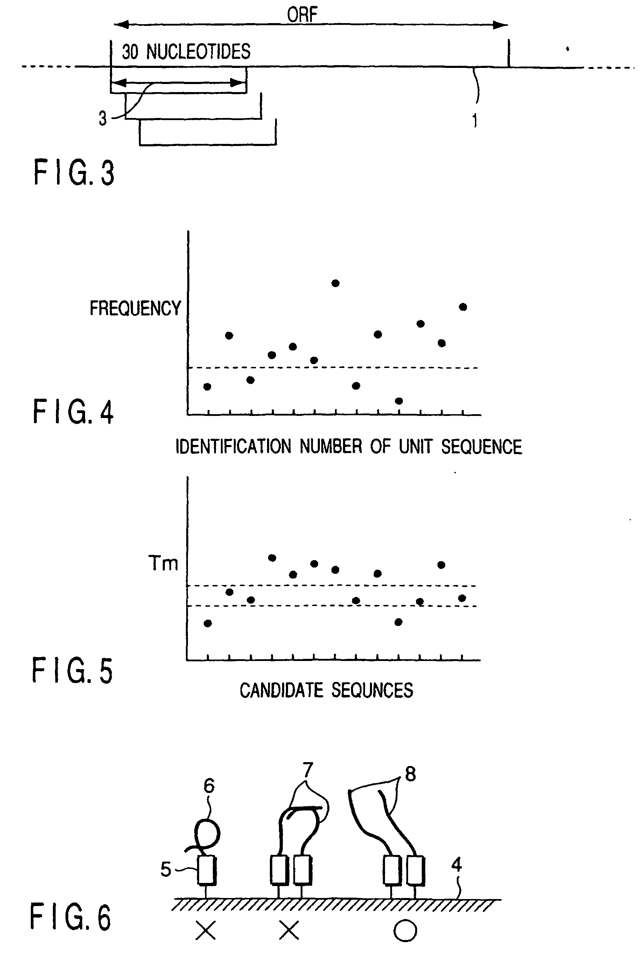

Using the figure of 4⁷ (= 16384) as the denominator, the occurrence frequency of each 7-tuple present on the entire genome is obtained (First calculation step). In this case, comparison can be readily made by using a graph as shown in FIG. 4 in which individual tuples are plotted on the lateral axis and their frequencies are plotted on the vertical axis. The calculation and graph-drawing mentioned above can be readily made by using a commercially-available computer. Note that the data as to frequencies of individual 7-tuples are stored in a memory.

As shown in FIG. 3, all the candidate sequences possibly used as an analytical sequence are completely listed, wherein the analytical sequence consists of nucleotides the number of which is larger than that of the 7-tuple by at least one nucleotide, preferably 10-50 nucleotides (e.g., 30 nucleotides), and is present on the ORF to be detected. With respect to each candidate sequence of 30-nucleotides, an index of the occurrence frequency is calculated on the basis of the occurrence frequency of the 7-tuple obtained in the first calculation step, as described below.

There are twenty four 7-tuples in the 30 nucleotides. Assuming that the twenty-four 7-tuples are numbered 1 to 24 sequentially from the side of the 5' terminal of the 30 nucleotides, and that the frequencies corresponding to the twenty-four 7 tuples calculated above are represented by p1, p2 ····· p24. In this case, the occurrence frequency index of the 30-necleotide analytical sequence can be calculated by multiplying the frequencies of the twenty-four 7 tuples with each other, as represented by p1 x p2 x ····· p24. The occurrence frequency index indicates how specifically a candidate sequence hybridizes with the ORF to be detected. The lower the value of the index, the higher the specificity. The occurrence frequency index is calculated with respect to all 30-nucleotide candidate sequences present on the target ORF. The candidate sequences are selected based on an appropriate threshold value of the index. The candidate sequences selected in this calculation step are referred to as "low occurrence frequency candidate sequence group". Note that the calculation and graph-drawing can be readily performed by a commercially available computer. Data of the occurrence frequency of individual 30 nucleotide partial sequences are stored in a memory.

A group of low occurrence frequency candidate sequences extracted in the above is evaluated for other conditions other than the occurrence frequency, that is, physicochemical conditions, thereby selecting a desired probe sequence. The probe sequence is not determined by the occurrence frequency alone. This is because the probe having a nucleotide sequence specific to a target sequence does not always form the hybrid efficiently. It is therefore preferable that each of low occurrence frequency candidate sequences be checked for thermal stability, as shown in FIGS. 5 and 6. First of all, Tm values are plotted on a graph shown in FIG. 5. Then, the low occurrence frequency candidate sequences within a predetermined range of Tm values are selected. The Tm values are calculated based on, for example, a SantaLucia parameter (John SantaLucia, Jr., Hatim T. Allawi, and P. Ananda Seneviratne "Improved nearest-neighbor parameters for predicting DNA duplex stability." Biochemistry 35, 3555-3562). The reason why the sequences having Tm values of the predetermined range are chosen is that the plurality of analytical nucleotide sequences which have specificities to the corresponding ORFs and satisfy the Tm requirement can hybridize with the ORFs simultaneously under the same temperature. The remaining low occurrence frequency candidate sequences which are not eliminated in the aforementioned selection step, are regarded as more potential candidate sequences, so that they are checked for the stability of a secondary structure formed within a molecule itself.

For example, as shown in FIG. 6, analytical nucleic acids are immobilized on a solid-phase support 4 with an appropriate linker molecule 5 interposed between them. When the construct is placed in a solution mixture containing a reactive substance such as a test sample, the stability of the molecular structure can be discussed as follows. A candidate probe 6 forms a loop called "auto-hybrid" in the molecule. A candidate sequence 7 partially forms an intermolecular hybridization with another analytical, sequence immobilized on the support 4. Since the secondary structures of these probes are stable, it may be difficult or impossible for them to form a desired hybrid with the target nucleic acid. Therefore, these probes capable of forming stable secondary structures are eliminated. As a result, a partial sequence 8 which is capable of readily hybridizing with a target under hybridization conditions, is selected as the most potential candidate sequence. The stability of the secondary structure can be calculated based upon the nucleotide sequence by use of an appropriate analytical software.

Finally, the most potential candidate sequences which are selected on the basis of the occurrence frequency and physicochemical conditions are further checked for their availability as the analytical sequence for identifying the ORF. The availability is checked by using a full length of the genome of Escherichia coli. More specifically, whether or not the selected analytical nucleotide sequence binds complementarily to an only one specific position of the genome is checked. The analysis for binding specificity is performed by using a computer. For example, a local binding map is first formed by a dynamic programming method and then the nucleotide sequences of the entire genome of Escherichia coli are compared to the map. In this manner, it can be verified that it is not feasible for the sequence to cause a mis-hybridization. The verification step may be also performed by calculating Hamming distance between the most potential candidates sequences and the nucleotide sequence of the entire genome of Escherichia coli. The nucleotide sequence passed the verification step is determined as the analytical nucleotide sequence.

When detection is performed based on hybridization using the analytical sequence determined in the above, a marker probe is prepared by binding a detectable marker substance to the oligo nucleic acid having the analytical sequence. The hybridization reaction can be performed in a predetermined manner in which the marker probe and a test sample are mixed together, and then, the hybridized marker substance is selectively measured. If a fluorescent substance such as FITC (Fluorescein Isothiocyanate) is employed as the marker substance, the detection can be readily made by an appropriate fluorescent detector. Further, by using a data processing means, quantitative or qualitative analysis can be automatically performed. In this case, it is possible to determine the presence or absence of the target nucleic acid molecule or a reaction amount on the basis of qualitative or quantitative measurement data (numerical value or image). The results of the gene analysis can be obtained by printing the result on a paper sheet as a report or displaying the result on a screen.

The hybridization reaction of nucleic acids is used not only for detecting a gene but also in a nucleic acid amplification reaction such as PCR, and furthermore, used in an identification reaction such as LCR (Ligase Chain Reaction).

The oligo nucleic acid having the analytical sequence designed according to the method of the present invention can be used as a primer in the PCR or as a probe in the LCR. Furthermore, plural types of analytical sequences may be appropriately applied to a single genome depending upon a principal of detection. The analytical oligo nucleic acid may be immobilized on a solid phase support such as microparticles, chip substrate, column, filter, test paper, well.

The present invention is not limited to aforementioned embodiments and may be modified in various ways on the basis of the gist of the present invention. For example, all steps explained in FIG. 1 may be automatically performed. In this case, it is sufficient that only the finally determined probe sequence is displayed or output. Depending upon the user's wish, it may be possible to omit the screen display or print-out of the graph for an occurrence frequency of the tuple, and data and graph with respect to a binding site of the analytical sequence on a target nucleic acid to be detected, and the Tm value and a secondary structure of the analytical sequence. Alternatively, individual calculation steps, calculation with respect to Tm value and the stability of the secondary structure, and conversion of the results into numerals or a graph may be performed by a computer, whereas evaluation including a final selection may be performed by a user on the basis of the numerical data or graph displayed on the screen display. In this case, the data extracted or selected herein may be input by using an input means such as a keyboard or mouse. In addition, the data obtained through various computation or calculations are not always necessary to be stored in a memory etc. In the automatic operation, various calculation data and the results of extraction and determination may be exchanged with various institutes such as hospitals, universities, examination centers by mutually transmitting them on an on-line network connecting these institute and a host computer.

Evaluation steps of the Tm value and the stability of the secondary structure may be performed in inverse order or at the same time. When the most preferable analytical nucleotide sequence is selected from the extracted low occurrence frequency candidate sequences under the condition in which a first preference is given to the physicochemical conditions, the analytical nucleotide sequence may be selected so as to include a tuple whose occurrence frequency is not the lowermost one.

In the method of the present invention can be applied to not only a genomic nucleotide sequence, but also expressed messenger RNA and cDNA (a copy of RNA), and further, artificially synthesized DNA may be used as a target. More specifically, the method of the present invention is used to design an analytical nucleotide sequence directed to a specific nucleotide sequence of any one of the aforementioned targets.

### Example

A PCR experiment was performed using primers designed by the method of the present invention for amplification of a mouse genes by PCR method, as described below.

The nucleotide sequences of all genes of a mouse (balb/c) were not elucidated. Therefore, the nucleotide sequence of a mouse (balb/c) registered in GenBank as of September 5, 1999 was used by assuming it as the entire nucleotide sequence of a mouse. Primers shown below were prepared for amplifying the DNA of a mouse.

| group | Gen Bank note | Name of Gene |
|---|---|---|
| 1 | 138444 | TGTP/Mg21 |
| 2 | m15525 | Lamini B1 |
| 3 | m18194 | Fibronectin |
| 4 | m35725 | Cu/Zn-SOD |
| 5 | m37030 | Diff6 |

The sequences of the primer obtained through calculation, calculated lengths of the amplified products, and Tm values are shown in a table below. Note that the primer sequence is written from the 5' end. It took two hours to perform calculation for obtaining the primer sequences under the following conditions. If the same calculation is performed without using the tuple method of the present invention, it takes 11.5 hours or more.

Computer used herein
- CPU:: Pentium III 500 MHz
- RAM:: 384 Mbyte
- OS:: Linux
- Compiler:: c⁺⁺

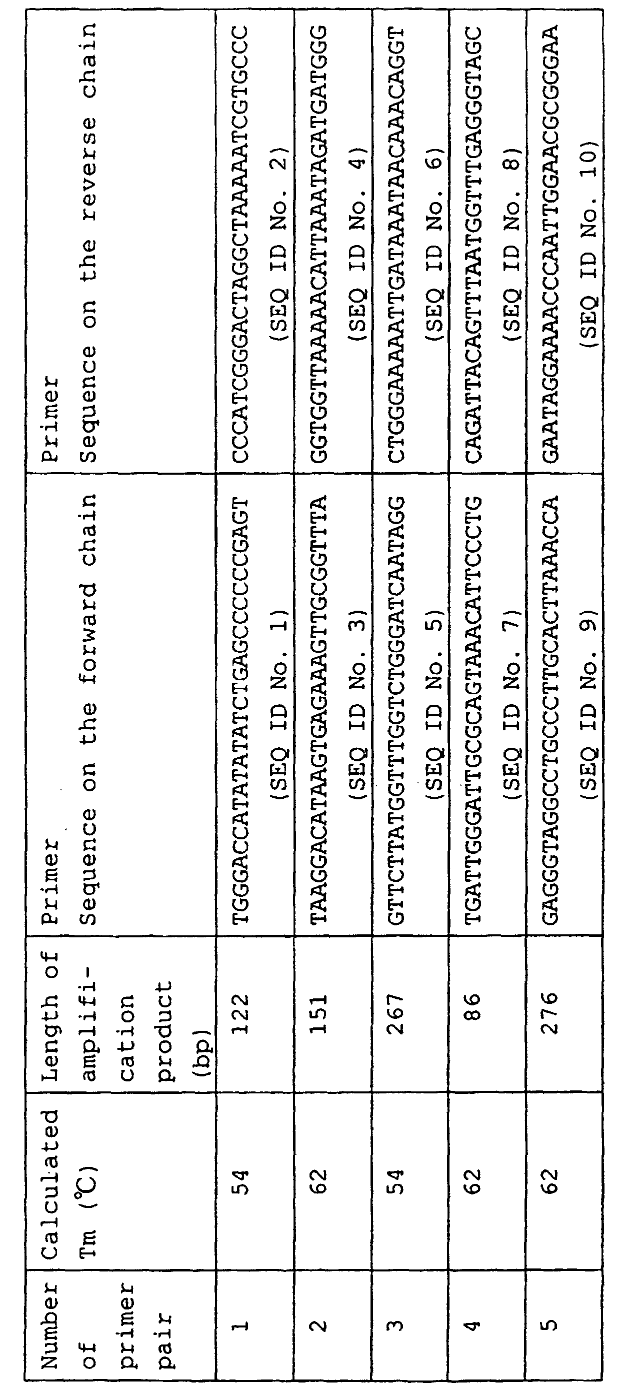

### PCR condition

| Reaction solution | Constituent per 50 µL |
|---|---|
| Template | manufactured by Clonetech. 0.4 µg of Genomic DNA extracted from a mouse (balb/c) liver |
| Enzyme manufactured by TaKaRa. | ExTaq 5 units |
| dNTP (mixture of dATP, dCTP, dGTP, dTTP) | 2.5 nmol for each |
| Buffer for ExTaq | manufactured by TaKaRa, Mg²⁺ concentration 2 mM) |
| Primer | 20 pmol for each |

### Temperature Cycle conditions for PCR

(1) 95°C 30 seconds
(2) 65°C 60 seconds
(3) 72°C 60 seconds

To improve stringency, the temperatures were set higher than required. Steps (2) and (3) were repeated 30 cycles.

### Electrophoresis conditions

gel: manufactured by FMC
Nusieve GTG agarose 4% TAE buffer
Voltage and time: 100V, 30 minutes

### Results

Amplified products by the PCR reaction were obtained with expected lengths.

As described in the above, according to the method of the present invention, it is possible to determine an analytical sequence which can always accurately hybridize with a target nucleotide sequence. Furthermore, according to the present invention, it is possible to quickly determine the analytical sequence. Furthermore, according to the present invention, the analytical sequence is determined step by step by combining relatively small-amounts of calculations without requiring a large calculation capacity. Therefore, a large-size computer is not required. Hence, determination of the analytical sequence can be simply performed by using an economically favorable computer for general use.

## Claims

1. A method of determining a nucleotide sequence of an analytical oligo nucleic acid, wherein said oligo nucleic acid is used for the analysis of nucleic acids, comprising the steps of:
listing all unit nucleotide sequences present on a target nucleic acid to be analyzed with a predetermined length which is shorter than the analytical oligo nucleic acid to be designed; and
extracting, based on the occurence frequency of the individual unit sequences listed, a nucleotide sequence containing a sequence occurring at a low frequency in the target nucleic acid from candidate sequences for analytical oligo nucleic acids, as an analytical sequence suitable for the analysis of the nucleotide sequence of the target nucleic acid.

2. The method according to claim 1, wherein the extraction step is performed by successively applying a plurality of different processes.

3. The method according to claim 1, wherein the extraction step further comprises a step of selecting the candidate sequences on the basis of the stability of the molecular structure of each oligo nucleic acid of the candidate sequences.

4. The method according to claim 3, wherein the stability of a molecular structure is thermal stability.

5. The method according to claim 3, wherein the stability of a molecular structure is measured by the melting temperature (TM) of the candidate sequences and/or by stability of an intramolecular secondary structure formed of the candidate sequences.

6. A method of determining a nucleotide sequence of an analytical oligo nucleic acid, wherein said oligo nucleic acid is used for the analysis of a nucleic acid sequence, comprising the steps of:
a first calculation step of calculating an occurence frequency of each of the n unit sequences occuring in a nucleotide sequence of a target nucleic acid to be analyzed on the basis of a value of 4ⁿ which corresponds to all of the n unit sequences formed of n nucleotide sequences (n is an integer of 2 or more);
a first extraction step of extracting a sequence having p nucleotides that is present in the nucleotide sequence of a target nucleic acid, said p is larger than n by m (m is an integer of 1 or more);
a second calculation step of extracting n unit sequences occuring in the candidate sequence extracted in the first extraction step and obtaining an occurrence frequency index of the candidate sequence in the nucleotide sequence of the target nucleic acid on the basis of the occurrence frequency of each of the n unit sequences obtained in the first calculation step; and
a second extraction step of selecting a single or plurality of candidate sequences having a low occurrence frequency index obtained in the second calculation step as potential candidate sequences.

7. The method according to claim 6, wherein said n is 5, 6, or 7.

8. The method according to claim 6, further comprising a third extraction step of selecting a candidate sequence having a low stability on the basis of the stability of a molecular structure of each of the oligo nucleic acid molecules formed by the potential candidate sequences.

9. The method according to claim 8, wherein the stability of a molecular structure is measured by the magnitude of the Tm value and/or by the stability of an intramolecular secondary structure.

10. The method according to claim 9, wherein, in said third extraction step, a sequence with the Tm value falling within a predetermined range and that forms an unstable secondary structure is selected from the potential candidate sequences.

11. The method according to any one of claims 1 to 10, wherein all necessary steps are sequentially performed by a computer.

12. A method, wherein the analytical oligo nucleid acid sequence according to any one of claims 1 to 11 is used for detecting a specific nucleotide sequence present in a nucleotide sequence of a nucleic acid by using an enzyme reaction which requires hybridization reactions of nucleic acids, or is used in a hybridization reaction of the nucleic acid.

## Patentansprüche

1. Verfahren zur Bestimmung einer Nukleotidsequenz einer analytischen Oligonukleinsäure, wobei die Oligonukleinsäure zur Analyse von Nukleinsäuren verwendet wird, umfassend die Schritte:
Auflisten aller Nukleotidsequenzeinheiten, die in der zu analysierenden target-Nukleinsäure mit vorbestimmter Länge, die kürzer als die zu gestaltende, analytische Oligonukleinsäure ist, vorhanden sind; und
Extraktion einer Nukleotidsequenz aus Kandidatensequenzen für analytische Oligonukleinsäuren, basierend auf der Auftrittshäufigkeit der aufgelisteten individuellen Sequenzeinheiten, wobei die Nukleotidsequenz eine Sequenz enthält, die mit geringer Häufigkeit in der target-Nukleinsäure auftritt, als eine analytische Sequenz, die für die Analyse der Nukleotidsequenz der target-Nukleinsäure geeignet ist.

2. Verfahren gemäß Anspruch 1, wobei der Extraktionsschritt durch sukzessive Anwendung einer Viel-zahl verschiedener Verfahrensschritte durchgeführt wird.

3. Verfahren gemäß Anspruch 1, wobei der Extraktionsschritt ferner einen Schritt der Selektion der Kandidatensequenzen, basierend auf der Stabilität der Molekularstruktur jeder Oligonukleinsäure der Kandidatensequenzen umfaßt.

4. Verfahren gemäß Anspruch 3, wobei die Stabilität einer Molekularstruktur thermische Stabilität bedeutet.

5. Verfahren gemäß Anspruch 3, wobei die Stabilität einer Molekularstruktur durch die Schmelztemperatur (TM) der Kandidatensequenzen und/oder durch die Stabilität einer intramolekularen Sekundärstruktur, die von den Kandidatensequenzen ausgebildet wird, gemessen wird.

6. Verfahren zur Bestimmung einer Nukleotidsequenz einer analytischen Oligonukleinsäure, wobei die Oligonukleinsäure zur Analyse einer Nukleinsäuresequenz verwendet wird, umfassend die Schritte:
einen ersten Rechenschritt zur Berechnung der Auftrittshäufigkeit jeder der n Sequenzeinheiten, die in einer Nukleotidsequenz einer zu analysierenden target-Nukleinsäure vorkommen, basierend auf einem Wert von 4ⁿ, der allen der n Sequenzeinheiten, die aus n Nukleotidsequenzen ausgebildet wurden, entspricht (n ist eine ganze Zahl von 2 oder mehr);
einen ersten Extraktionsschritt zur Extraktion einer Sequenz, die p Nukleotide in der Nukleotidsequenz einer target-Nukleinsäure aufweist, wobei p größer als n mal m ist (m ist eine ganze Zahl von 1 oder mehr);
einen zweiten Rechenschritt zur Extraktion von n Sequenzeinheiten, die in der Kandidatensequenz, die im ersten Extraktionsschritt extrahiert wurde, auftreten und Erhalten eines Auftrittshäufigkeitsindexes der Kandidatensequenz in der Nukleotidsequenz der target-Nukleinsäure, basierend auf der Auftrittshäufigkeit jeder
der n Sequenzeinheiten, die im ersten Rechenschritt erhalten wurden; und
einen zweiten Extraktionsschritt zur Selektion einer einzelnen oder mehreren Kandidatensequenzen, die einen niedrigen Auftrittshäufigkeitsindex aufweisen, der im zweiten Rechenschritt erhalten wurde als mögliche Kandidatensequenzen.

7. Verfahren gemäß Anspruch 6, wobei n 5, 6 oder 7 ist.

8. Verfahren gemäß Anspruch 6, ferner umfassend einen dritten Extraktionsschritt zur Selektion einer Kandidatensequenz, die eine niedrige Stabilität basierend auf der Stabilität einer Molekularstruktur jeder der Oligonukleinsäuremoleküle aufweist, welche von möglichen Kandidatensequenzen ausgebildet wurden.

9. Verfahren gemäß Anspruch 8, wobei die Stabilität einer Molekularstruktur durch die Höhe des Tm-Werts und/oder durch die Stabilität einer intramolekularen Sekundärstruktur gemessen wird.

10. Verfahren gemäß Anspruch 9, wobei, im dritten Extraktionsschritt, eine Sequenz, mit einem in einen vorher bestimmten Bereich fallenden Tm-Wert und die eine instabile Sekundärstruktur ausbildet, aus den möglichen Kandidatensequenzen selektiert wird.

11. Verfahren gemäß irgendeinem der Ansprüche 1 bis 10, wobei alle notwendigen Schritte aufeinanderfolgend von einem Computer durchgeführt werden.

12. Verfahren, wobei die analytische Oligonukleinsäuresequenz gemäß einem der Ansprüche 1 bis 11 zur Detektion einer spezifischen Nukleinsäuresequenz verwendet wird, die in einer Nukleotidsequenz einer Nukleinsäure vorhanden ist, indem eine Enzymreaktion, die Hybridisierungsreaktionen der Nukleinsäuren erforderlich macht, verwendet wird oder indem sie in einer Hybridisierungsreaktion der Nuklein-säure verwendet wird.

## Revendications

1. Procédé de détermination d'une séquence nucléotidique d'un acide oligonucléique analytique, Ledit acide oligonucléique étant utilisé pour l'analyse d'acides nucléiques, comprenant les étapes consistant à :
lister toutes les séquences nucléotidiques unitaires présentes sur un acide nucléique cible à analyser avec une longueur prédéterminée qui est plus courte que l'acide oligonucléique analytique à concevoir ; et
extraire, sur la base de la fréquence d'occurrence des séquences unitaires individuelles listées, une séquence nucléotidique contenant une séquence apparaissant à une fréquence faible dans l'acide nucléique cibLe, à partir de séquences candidates pour des acides oligonucléiques analytiques, comme séquence analytique adaptée à l'analyse de la séquence nucléotidique de l'acide nucléique cible.

2. Procédé selon la revendication 1, dans lequel l'étape d'extraction est effectuée en appliquant successivement une pluralité de procédures différentes.

3. Procédé selon la revendication 1, dans lequel l'étape d'extraction comprend en outre une étape de sélection de séquences candidates sur la base de la stabilité de la structure moléculaire de chaque acide oligonucléique des séquences candidates.

4. Procédé selon la revendication 3, dans lequel la stabilité d'une structure moléculaire est une stabilité thermique.

5. Procédé selon la revendication 3, dans lequel la stabilité d'une structure moléculaire est mesurée par la température de fusion (T_{f}) des séquences candidates et/ou par la stabilité d'une structure secondaire intramoléculaire formée des séquences candidates.

6. Procédé de détermination d'une séquence nucléotidique d'un acide oligonucléique analytique, ledit acide oligonucléique étant utilisé pour l'analyse d'une séquence d'acide nucléique, comprenant les étapes constituées par :
une première étape de calcul pour calculer la fréquence d'occurrence de chacune des n séquences unitaires apparaissant dans une séquence nucléotidique d'un acide nucléique cible à analyser rapportée à une valeur de 4ⁿ qui correspond à toutes les n séquences unitaires formées de n séquences nucléotidiques (n est un entier valant 2 ou plus) ;
une première étape d'extraction pour extraire une séquence ayant p nucléotides qui est présente dans la séquence nucléotidique d'un acide nucléique cible, ledit p étant plus grand que n par m (m est un entier valant 1 ou plus) ;
une seconde étape de calcul pour extraire n séquences unitaires apparaissant dans la séquence candidate extraite à la première étape d'extraction et obtenir un indice de fréquence d'occurrence de la séquence candidate dans la séquence nucléotidique de l'acide nucléique cible sur la base de la fréquence d'occurrence de chacune des n séquences unitaires obtenue à la première étape de calcul ; et
une seconde étape d'extraction pour sélectionner une seule ou une pluralité de séquences candidates ayant un indice de fréquence d'occurrence faible obtenu à la seconde étape de calcul comme séquences candidates potentielles.

7. Procédé selon la revendication 6, dans lequel ledit n vaut 5, 6, ou 7.

8. Procédé selon la revendication 6, comprenant en outre une troisième étape d'extraction pour sélectionner une séquence candidate ayant une faible stabilité sur la base de la stabilité d'une structure moléculaire de chacune des molécules d'acides oligonucléiques formées par les séquences candidates potentielles.

9. Procédé selon la revendication 8, dans lequel la stabilité d'une structure moléculaire est mesurée par la grandeur de la valeur de T_{f} et/ou par la stabilité d'une structure secondaire intramoléculaire.

10. Procédé selon la revendication 9, dans lequel, à ladite troisième étape d'extraction, une séquence avec une valeur de T_{f} se situant à l'intérieur d'une fourchette prédéterminée et qui forme une structure secondaire instable est choisie parmi les séquences candidates potentielles.

11. Procédé selon l'une quelconque des revendications 1 à 10, dans lequel toutes les étapes nécessaires sont effectuées séquentiellement par un ordinateur.

12. Procédé dans lequel la séquence de l'acide oligonucléique analytique selon l'une quelconque des revendications 1 à 11 est utilisée pour détecter une séquence nucléotidique spécifique présente dans une séquence nucléotidique d'un acide nucléique à l'aide d'une réaction enzymatique qui nécessite des réactions d'hybridation d'acides nucléiques, ou est utilisée dans un réaction d'hybridation de l'acide nucléique.
